# FASCICULE DE BREVET EUROPEEN

(11) **EP 2 486 059 B1**
(45) Date de publication et mention de la délivrance du brevet: **11.11.2015**
(21) Numéro de dépôt: 10785516.5
(22) Date de dépôt: 08.10.2010
(51) Int. Cl.: C07K 16/28

(54) **LIGANDS MONOVALENTS DU RÉCEPTEUR CD28 HUMAIN**
MONOVALENTE LIGANDEN DES MENSCHLICHEN CD28-REZEPTORS
MONOVALENT LIGANDS OF THE HUMAN CD28 RECEPTOR

(30) Priorité: 09.10.2009 FR 0904866
(43) Date de publication de la demande: 15.08.2012
(73) Titulaire: Effimune, 44035 Nantes Cedex (FR); Institut National de la Santé et de la Recherche Médicale, 75013 Paris (FR)
(72) Inventeur: VANHOVE, Bernard, F-44400 Rezé (FR); MARY, Caroline, F-44680 Sainte Pazanne (FR)
(74) Mandataire: Marcadé, Véronique
(86) Numéro de dépôt international: PCT/IB2010/054562
(87) Numéro de publication internationale: WO 2011/042891

(56) Documents cités:
- WO-A1-02/47721
- WO-A1-2009/018441
- WO-A2-02/051871
- NUNES J ET AL: "CD28 MABS WITH DISTINCT BINDING PROPERTIES DIFFER IN THEIR ABILITY TO INDUCE T CELL ACTIVATION: ANALYSIS OF EARLY AND LATE ACTIVATION EVENTS", INTERNATIONAL IMMUNOLOGY, OXFORD UNIVERSITY PRESS, GB, vol. 5, no. 3, 1 janvier 1993 (1993-01-01) , pages 311-315, XP001024214, ISSN: 0953-8178
- VANHOVE B ET AL: "Selective blockade of CD28 and not CTLA-4 with a single-chain Fv-alpha1-antitrypsin fusion antibody", BLOOD, AMERICAN SOCIETY OF HEMATOLOGY, US LNKD- DOI:10.1182/BLOOD-2002-08-2480, vol. 102, no. 2, 15 juillet 2003 (2003-07-15), pages 564-570, XP002334754, ISSN: 0006-4971
- TAN P ET AL: "INDUCTION OF ALLOANTIGEN-SPECIFIC HYPORESPONSIVENESS IN HUMAN T LYMPHOCYTES BY BLOCKING INTERACTION OF CD28 WITH ITS NATURAL LIGANDB7/BB1", THE JOURNAL OF EXPERIMENTAL MEDICINE, ROCKEFELLER UNIVERSITY PRESS, US LNKD- DOI:10.1084/JEM.177.1.165, vol. 177, no. 1, 1 janvier 1993 (1993-01-01), pages 165-173, XP000572919, ISSN: 0022-1007

## Description

La présente invention est relative à la sélection de ligands monovalents du récepteur CD28 humain, permettant de bloquer l'interaction CD28/B7 sans activer CD28.

L'activation des lymphocytes T nécessite un signal activateur, induit par la reconnaissance par les récepteurs T (TCR) de l'antigène associé avec le complexe majeur d'histocompatibilité (CMH) de classe I ou II et présenté par les cellules présentatrices de l'antigène (CPAg). Cette activation n'entraîne cependant la prolifération des cellules T et la sécrétion de cytokines immunomodulatrices spécifiques (telles que l'interleukine 2, l'interféron gamma ou l'interleukine 4), que si d'autres systèmes de co-stimulation T sont également activés.

L'un des systèmes les plus importants de régulation de l'activation des lymphocytes T est le système moléculaire B7/CD28/CTLA4. Ce système joue par exemple un rôle essentiel dans les mécanismes du rejet de greffe [WOODWARD et al., Transplantation, 66, 14-20, (1998)]. Les molécules B7.1 (CD80) et B7.2 (CD86), collectivement désignées ci-après sous le terme « B7 », qui sont portées par les CPAgs peuvent activer le récepteur CD28 ainsi que le récepteur CTLA4 des lymphocytes T. L'activation du CD28 délivre au lymphocyte T un signal positif stimulant la cellule ; en revanche, l'activation du CTLA4 délivre un signal négatif conduisant à une non-réponse (anergie) [FALLARINO et al., J. Exp. Med., 188, 205-210, (1998)].

Les lymphocytes T au repos expriment une quantité importante de CD28, et très peu de CTLA4. Lors d'un premier contact cognitif entre une CPAg et un lymphocyte T, l'interaction CD28/B7 est privilégiée, ce qui active la cellule. Ce n'est que plusieurs heures après l'initiation de l'activation, du fait de l'augmentation de l'expression membranaire de CTLA4 dont l'affinité pour B7 est 5 à 10 fois supérieure à celle du CD28, que l'interaction B7/CD28 se déplace au profit d'une interaction B7/CTLA4.

L'inhibition sélective du signal agoniste délivré à la cellule T par le CD28 en laissant intact le système antagoniste constitué par le couple CTLA4/B7, par l'intermédiaire d'un blocage spécifique de l'interaction CD28/B7 peut permettre de prévenir l'activation des lymphocytes T, et ainsi favoriser l'induction de la tolérance dans le cas de transplantation d'organes, ainsi que dans le cadre du traitement de maladies auto-immunes.

Cette inhibition sélective peut être obtenue en ciblant le récepteur CD28 des cellules T, notamment à l'aide d'un anticorps dirigé contre CD28.

Cependant, tous les anticorps ne possèdent pas la même capacité à inhiber sélectivement l'interaction CD28/B7. En effet, une grande partie d'entre eux, lorsqu'ils sont utilisés sous leur forme native divalente, entraînent la dimérisation de CD28, qui provoque son activation, et s'ils sont utilisés sous forme de fragments monovalents, qui n'entraînent pas la dimérisation de CD28, ne présentent plus d'effet inhibiteur de l'interaction CD28/B7. D'autres anticorps anti-CD28, dénommés anticorps superagonistes, sont capables d'activer les lymphocytes T, même en l'absence des signaux d'activation induits par l'interaction TCR/CMH. Ces différentes propriétés des anticorps anti-CD28 ont été attribuées à leur capacité à reconnaître différentes régions de CD28 (NUNES et al., Int. Immunol., 5, 311-315, 1993 ; LUHDER et al., J Exp Med 197, 955-66 2003).

Dans l'article de NUNES *et al.* (1993, précité), différents anticorps monoclonaux anti-CD28 ont été utilisés pour analyser les relations structure-fonction avec la molécule CD28. Parmi ces anticorps, un sous-groupe composé de CD28.1, CD28.3 et CD28.5 a été identifié comme particulièrement intéressant. En effet, ces anticorps présentent des profils d'inhibition vis-à-vis de CD28 pratiquement identiques.

Au cours de travaux précédent, l'équipe des Inventeurs a sélectionné parmi des anticorps monoclonaux anti-CD28 décrits par NUNES *et al*. (1993, précité), un anticorps dénommé CD28.3 dont les fragments monovalents possèdent la capacité d'inhiber l'interaction CD28/B7. L'hybridome produisant l'anticorps CD28.3 a été déposé auprès de la CNCM sous le numéro I-2582, et est décrit dans la Demande PCT WO 02/051871. Cette demande internationale divulgue des fragments monovalents de l'anticorps CD28.3 qui présentent une affinité suffisante pour être utilisables *in vitro* ou *in vivo* afin de bloquer le récepteur CD28 sans induire son activation.

Les Inventeurs ont maintenant caractérisé l'épitope reconnu par l'anticorps CD28.3, ce qui permet d'identifier, d'autres ligands monovalents de CD28 possédant les mêmes propriétés que les fragments monovalents issus de CD28.3. Cet épitope est un épitope conformationnel, localisé dans les domaines C, D, E, F de la molécule CD28.

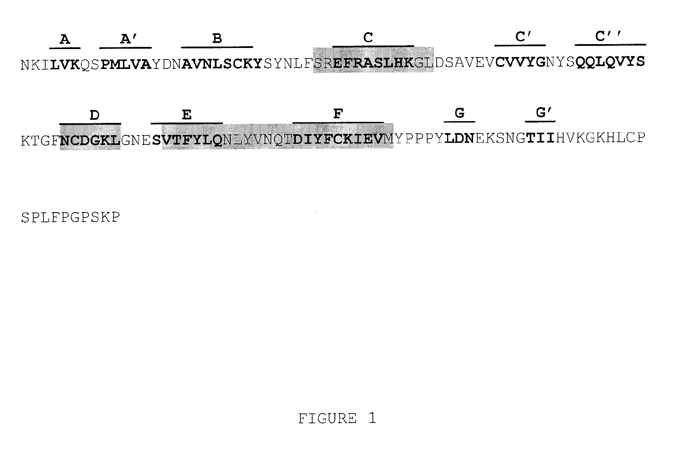

La séquence d'une portion du récepteur CD28 humain, contenant l'épitope reconnu par l'anticorps CD28.3, est représentée sur la Figure 1. Les différents domaines A, A' B, C, C' C", E, F, G et G', tels que définis par EVANS et al. (Nat Immunol., 6(3):271-9, 2005) sont indiqués au dessus de la séquence. Les régions constituant l'épitope sont surlignées en gris. Cette séquence est également représentée dans la liste de séquences en annexe sous l'identifiant SEQ ID NO: 1.

Est décrit ici un ligand monovalent du récepteur CD28 humain, capable de bloquer sélectivement l'interaction CD28/B7 sans activer le récepteur CD28, caractérisé en ce qu'il reconnaît un épitope formé par les portions de la séquence SEQ ID NO: 1 ayant les séquences suivantes :
SREFRASLHKGL (SEQ ID NO: 2) :
NCDGKL (SEQ ID NO: 3) ;
VTFYLQNLYVNQTDIYFCKIEVM (SEQ ID NO: 4),
à l'exception d'un ligand monovalent ayant les CDRs de la chaîne lourde et les CDRs de la chaîne légère de l'immunoglobuline CD28.3.

On définit ici comme « ligand monovalent » tout ligand du récepteur CD28 humain possédant un seul site de liaison audit récepteur CD28. Ledit ligand est de préférence une protéine ; il peut s'agir par exemple d'un fragment monovalent d'un anticorps anti-CD28, ou bien d'une anticaline (SKERRA et al., FEBS J., 275(11),:2677-83, 2008).

Selon un mode de réalisation préféré, ledit ligand monovalent est une protéine comprenant les CDRs de la chaîne lourde et les CDRs de la chaîne légère d'un anticorps anti-CD28 reconnaissant l'épitope défini ci-dessus. Il peut s'agir notamment d'un fragment monovalent (Fv, Fab ou scFv) dudit anticorps, ou d'une protéine recombinante associant ledit fragment monovalent avec un polypeptide hétérologue, comme décrit par exemple dans la Demande PCT WO 02/051871 pour les fragments monovalents de l'anticorps CD28.3.

La présente invention a pour objet un procédé d'obtention d'un ligand monovalent du récepteur CD28 humain capable de bloquer sélectivement l'interaction CD28/B7 sans activer le récepteur CD28, caractérisé en ce qu'il comprend la mise en présence de ligands monovalents du récepteur CD28 humain avec un polypeptide ayant la séquence du récepteur CD28 humain ou d'un fragment de celui-ci comprenant les séquences SEQ ID NO: 2, SEQ ID NO: 3 et SEQ ID NO: 4, et la sélection des ligands reconnaissant un épitope formé par lesdites séquences SEQ ID NO: 2, SEQ ID NO: 3, et SEQ ID NO: 4.

Est décrit également ici toute molécule d'acide nucléique codant un ligand monovalent du récepteur CD28 humain, ainsi que tout vecteur recombinant, notamment tout vecteur d'expression, comprenant ladite molécule d'acide nucléique, et toute cellule-hôte transformée par ledit vecteur recombinant.

Des molécules d'acide nucléique peuvent avantageusement comprendre, outre une séquence codant une protéine telle que décrite plus haut, une séquence codant un peptide signal permettant la sécrétion de ladite protéine ; elles peuvent aussi comprendre une ou plusieurs séquence(s) codant un ou plusieurs peptide(s) marqueur(s) permettant la détection et/ou facilitant la purification de ladite protéine.

Des vecteurs d'expression comprennent au moins une séquence d'acide nucléique codant une protéine telle que décrite plus haut, associée à des éléments de contrôle de la transcription et de la traduction actifs dans la cellule-hôte choisie. Des vecteurs utilisables pour la construction de vecteurs d'expression sont connus en eux-mêmes, et seront choisis notamment en fonction de la cellule-hôte que l'on souhaite utiliser.

Des cellules-hôtes peuvent être des cellules procaryotes ou eucaryotes. Parmi les cellules eucaryotes utilisables, on citera en particulier des cellules végétales, des cellules de levure, telles que *Saccharomyces,* des cellules d'insecte, telles que les cellules de *Drosophila,* ou de *Spodoptera* et des cellules de mammifères telles que les cellules HeLa, CHO, 3T3, C127, BHK, COS, etc...

La construction de vecteurs d'expression, et la transformation des cellules-hôtes peuvent être effectuées par les techniques classiques de biologie moléculaire.

Des ligands monovalents du récepteur CD28 humain peuvent notamment être utilisées, de la même manière que les protéines monovalentes dérivées de CD28.3 décrites dans la Demande PCT WO 02/051871, pour l'obtention de médicaments immunosuppresseurs, bloquant sélectivement les phénomènes d'activation des cellules T impliquant le récepteur CD28.

Ces médicaments sont utilisables dans toutes les pathologies dépendantes des lymphocytes T.

Il s'agit essentiellement du rejet de greffe, de la maladie du greffon contre l'hôte, des maladies auto-immunes à médiation lymphocytaire T, telles que le diabète de type I, ou la sclérose en plaques, et de l'hypersensibilité de type IV, qui intervient dans les phénomènes allergiques ainsi que dans la pathogenèse de maladies inflammatoires chroniques suivant une infection par un agent pathogène (notamment lèpre, tuberculose, leishmaniose, listérose, etc.). Il peut s'agir également de maladies telles que le myélome, dans lesquelles des lymphocytes connaissent une prolifération dérégulée et que celle-ci est dépendante d'un signal transmis par CD28 (BAHLIS et al., Blood., 109(11):5002-10, 2007).

La présente invention sera mieux comprise à l'aide du complément de description qui va suivre, qui se réfère à un exemple illustrant l'identification de l'épitope reconnu par l'anticorps CD28.3.

### EXEMPLE : IDENTIFICATION DE L'EPITOPE RECONNU PAR L'ANTICORPS CD28.3.

Pour la détermination de l'épitope reconnu par l'anticorps CD28.3, des fragments Fab de CD28.3 ont été mis en présence de CD28 humain (R&D Systems, Lille, France) immobilisé sur Sepharose®. Les complexes immuns ont été réduits et alkylés par incubation pendant 1 heure en présence d'iodoacétamide (55mM), avant l'addition de chymotrypsine (1 mg/50mg d'anticorps lié) suivie d'incubation pendant 4h à 18-25°C. Le Sepharose® a ensuite été lavé avec 25mM de carbonate d'ammonium, puis 50mM glycine, pH 2,5. Les peptides élués ont ensuite été concentrés sur une matrice C18 et analysés par spectrométrie de masse MALDI-TOF/TOF, pour établir la carte peptidique massique.

Les résultats de cette analyse montrent que les fragments protégés de la protéolyse par la chymotrypsine sont situés dans les boucles C-D-E-F des molécules CD28, et constituent donc un épitope conformationnel. La séquence de la région du récepteur CD28 où est localisé cet épitope est représentée sur la Figure 1, et les séquences des fragments peptidiques qui forment l'épitope, ainsi que leur localisation par rapport aux domaines du CD28 humain sont listées dans le Tableau I ci-dessous.

**Tableau I**

| Domaine du CD28 humain | Séquence du peptide protégé de la protéolyse. |
|---|---|
| C | SREFRASLHKGL (SEQ ID NO: 2) |
| D | NCDGKL (SEQ ID NO: 3) |
| E/F | VTFYLQNLYVNQTDIYFCKIEVM (SEQ ID NO: 4) |

## Revendications

1. Procédé de sélection d'un ligand monovalent du récepteur CD28 humain, capable de bloquer sélectivement l'interaction CD28/B7 sans activer le récepteur CD28, **caractérisé en ce qu'**il comprend la mise en présence de ligands monovalents du récepteur CD28 humain avec un polypeptide ayant la séquence du récepteur CD28 humain ou d'un fragment de celui-ci comprenant les séquences SEQ ID NO: 2, SEQ ID NO: 3 et SEQ ID NO: 4, et la sélection d'un ligand reconnaissant un épitope formé par les séquences SEQ ID NO: 2, SEQ ID NO: 3, et SEQ ID NO: 4.

2. Procédé selon la revendication 1, **caractérisé en ce que** lesdits ligands sont des fragments monovalents d'anticorps anti-CD28 humain.

## Patentansprüche

1. Verfahren zur Auswahl eines monovalenten Liganden des humanen CD28-Rezeptors, der fähig ist, selektiv die CD28/B7-Wechselwirkung zu blockieren, ohne den CD28-Rezeptor zu aktivieren, **dadurch gekennzeichnet, dass** es das Inkontaktbringen von monovalenten Liganden des humanen CD28-Rezeptors mit einem Polypeptid, das die Sequenz des humanen CD28-Rezeptors oder eines Fragments desselben, umfassend die Sequenzen SEQ ID NO:2, SEQ ID NO:3 und SEQ ID NO:4, hat, und die Auswahl eines Liganden, der ein Epitop erkennt, das durch die Sequenzen SEQ ID NO:2, SEQ ID NO:3 und SEQ ID NO:4 gebildet wird, umfasst.

2. Verfahren gemäß Anspruch 1, **dadurch gekennzeichnet, dass** die Liganden monovalente Fragmente von humanem Anti-CD28-Antikörper sind.

## Claims

1. A method for selecting a monovalent ligand of the human CD28 receptor, capable of selectively blocking the CD28/B7 interaction without activating the CD28 receptor, **characterized in that** it comprises bringing monovalent ligands of the human CD28 receptor into contact with a polypeptide which has the sequence of the human CD28 receptor or a fragment thereof comprising the sequences SEQ ID NO: 2, SEQ ID NO: 3 and SEQ ID NO: 4, and selecting a ligand which recognises an epitope formed by the sequences SEQ ID NO: 2, SEQ ID NO: 3, and SEQ ID NO: 4.

2. The method as claimed in claim 1, **characterized in that** said ligands are monovalent fragments of the human anti-CD28 antibody.


### SEQUENCE LISTING

<110> TcL Pharma INSTITUT NATIONAL DE LA SANTE ET DE LA RECHERCHE MEDICALE (INSERM) VANHOVE, Bernard MARY, Caroline
<120> LIGANDS MONOVALENTS DU RECEPTEUR CD28 HUMAIN
<130> MJP11/F2173-2WO
<150> FR0904866
   <151> 2009-10-09
<160> 4
<170> PatentIn version 3.5
<210> 1
   <211> 134
   <212> PRT
   <213> Homo sapiens
<400> 1
   Asn Lys Ile Leu Val Lys Gln Ser Pro Met Leu Val Ala Tyr Asp Asn
   1               5                   10                  15
   Ala Val Asn Leu Ser Cys Lys Tyr Ser Tyr Asn Leu Phe Ser Arg Glu
               20                  25                  30
   Phe Arg Ala Ser Leu His Lys Gly Leu Asp Ser Ala Val Glu Val Cys
           35                  40                  45
   Val Val Tyr Gly Asn Tyr Ser Gln Gln Leu Gln Val Tyr Ser Lys Thr
       50                  55                  60
   Gly Phe Asn Cys Asp Gly Lys Leu Gly Asn Glu Ser Val Thr Phe Tyr
   65                  70                  75                  80
   Leu Gln Asn Leu Tyr Val Asn Gln Thr Asp Ile Tyr Phe Cys Lys Ile
                   85                  90                  95
   Glu Val Met Tyr Pro Pro Pro Tyr Leu Asp Asn Glu Lys Ser Asn Gly
               100                 105                 110
   Thr Ile Ile His Val Lys Gly Lys His Leu Cys Pro Ser Pro Leu Phe
           115                 120                 125
   Pro Gly Pro Ser Lys Pro
       130
<210> 2
   <211> 12
   <212> PRT
   <213> Homo sapiens
<400> 2
   Ser Arg Glu Phe Arg Ala Ser Leu His Lys Gly Leu
   1               5                   10
<210> 3
   <211> 6
   <212> PRT
   <213> Homo sapiens
<400> 3
   Asn Cys Asp Gly Lys Leu
   1               5
<210> 4
   <211> 23
   <212> PRT
   <213> Homo sapiens
<400> 4
   Val Thr Phe Tyr Leu Gln Asn Leu Tyr Val Asn Gln Thr Asp Ile Tyr
   1               5                   10                  15
   Phe Cys Lys Ile Glu Val Met
               20